# EUROPEAN PATENT APPLICATION

(11) **EP 4 324 934 A1**
(43) Date of publication of application: **21.02.2024**
(21) Application number: 22787551.5
(22) Date of filing: 13.04.2022
(51) Int. Cl.: C12Q 1/6851, C12M 1/36

(54) **FLUORESCENCE QUANTITATIVE PCR PROCESSING METHOD AND SYSTEM**

(30) Priority: 15.04.2021 CN 202110410842
(71) Applicant: Zhuhai Shineway Hi-Tech Biotechnology Co., Ltd, Zhuhai, Guangdong 519000 (CN)
(72) Inventor: SONG, Qi, Zhuhai, Guangdong 519000 (CN); GAO, Yibo, Zhuhai, Guangdong 519000 (CN); LIU, Yiteng, Zhuhai, Guangdong 519000 (CN); WEN, Weijia, Zhuhai, Guangdong 519000 (CN)
(74) Representative: Murgitroyd & Company
(86) International application number: PCT/CN2022/086547
(87) International publication number: WO 2022/218329

(57) **Abstract**

The present invention provides a fluorescent quantitative PCR treatment method and system. The method comprises: amplifying a sample to be tested; controlling a light source to emit light with multiple different wavelengths, wherein the emission time of the light with different wavelengths is different; controlling a shooting device to shoot according to the emission time of the light with different wavelengths, wherein objects shot by the shooting device are fluorescence emitted from the sample to be tested after the amplification of the sample to be tested excited by the light with different wavelengths, and the fluorescence reaches the shooting device through the same channel; and conducting analysis according to images shot by the shooting device.

## Description

The present invention is proposed based on a Chinese patent application with application number of 202110410842.4 and application date of April 15, 2021, and claims the priority of the Chinese patent application, the disclosures of which are hereby incorporated by reference.

### Technical Field

The present invention relates to the technical field of biological nucleic acid detection, and particularly relates to a fluorescent quantitative PCR treatment method and system.

### Background

As a molecular biology technology that can amplify a large number of specific DNA fragments in vitro in a short time, polymerase chain reaction (PCR) has played an important role in the fields of biomedical monitoring and disease control and screening. With the increase of the demand for qualitative and quantitative DNA detection, the real-time fluorescent quantitative PCR technology has been favored by people because it can detect viruses with accuracy, high sensitivity, strong specificity, simplicity and quantification. It mainly introduces specific probes and dynamic monitoring of fluorescence signals to realize real-time quantitative detection of the PCR process.

The PCR technology that uses multiplex PCRs for adding multiple pairs of specific primers into a reaction system and amplifies multiple target fragments for multiple templates has the characteristics of high accuracy, time-saving, cost reduction and efficiency increase and can significantly improve sensitivity, specificity and the anti-interference performance of fluorescent quantitative PCR detection.

However, the detection instrument under development currently has complex structure, large volume, high cost and particularly high performance requirements for optical elements, thereby leading to the almost impossibility of multiple PCR fluorescence detection.

### Summary

The purpose of embodiments of the present invention is to provide a fluorescent quantitative PCR treatment method and system to at least solve the problem of high cost or even impossibility of realization caused by the complicated design of multiple PCR fluorescence detection instruments in the related art.

The present invention adopts the following technical solution:
In one aspect, the present invention provides a fluorescent quantitative PCR treatment method, comprising: amplifying a sample to be tested; controlling a light source to emit light with multiple different wavelengths, wherein the emission time of the light with different wavelengths is different; controlling a shooting device to shoot according to the emission time of the light with different wavelengths, wherein objects shot by the shooting device are fluorescence emitted from the sample to be tested after the amplification of the sample to be tested excited by the light with different wavelengths, and the fluorescence reaches the shooting device through the same channel; and conducting analysis according to images shot by the shooting device.

Further, the light with different wavelengths is reflected onto the amplified sample to be tested at a first predetermined angle; the fluorescence is reflected onto the shooting device at a second predetermined angle; and the first predetermined angle is the same as or different from the second predetermined angle.

Further, an incident angle at which the light with different wavelengths is incident into the sample to be tested after reflected is determined according to an inclination angle of the light source and a distance between the light source and a microfluidic chip where the sample to be tested is placed.

Further, the method further comprises: after the light source emits the light with multiple different wavelengths, before the light with multiple different wavelengths reaches the sample to be tested, conducting first bandpass filtering on the light with multiple different wavelengths, wherein the first bandpass filtering is used for filtering the light with other wavelengths except the multiple wavelengths.

Further, the method further comprises: before the fluorescence reaches the shooting device, conducting second bandpass filtering and/or convergence on the fluorescence, wherein the second bandpass filtering is used for filtering the light with other wavelengths except the wavelength of the fluorescence, and the convergence is used for focusing the fluorescence to a photosurface of the shooting device.

Further, the method further comprises: under the same wavelength light source, simultaneously collecting the fluorescence intensity emitted by the sample to be tested excited respectively by a matched signal receiving bandwidth and a crosstalk signal receiving bandwidth, and counting the collected fluorescence intensity to obtain the rule of the fluorescence intensity; and in the crosstalk bandwidth, using a crosstalk signal as background light, and setting a fluorescence signal threshold according to the rule to eliminate the fluorescence crosstalk.

Further, the light with multiple different wavelengths comprises three or more kinds of light.

In another aspect, the present invention provides a fluorescent quantitative PCR treatment system, comprising a light excitation module for emitting light with multiple different wavelengths, wherein the emission time of the light with different wavelengths is different; a microfluidic chip module for amplifying the sample to be tested, wherein the fluorescence emitted from the sample to be tested after the amplification of the sample to be tested excited by the light with different wavelengths is transmitted to the shooting device, and the shooting device is used for shooting the fluorescence according to the emission time of the light with different wavelengths; and a single channel, wherein the fluorescence emitted from the sample to be tested after the amplification of the sample to be tested excited by light with different wavelengths reaches the shooting device through the single channel.

Further, the system further comprises: a first reflection device for reflecting the light with different wavelengths onto the amplified sample to be tested at a first predetermined angle; and a second reflection device for reflecting the fluorescence onto the shooting device at a second predetermined angle, wherein the first reflection device and the second reflection device are the same reflection device or different reflection devices.

Further, the system further comprises: a first bandpass filtering device arranged between the light excitation module and the sample to be tested and used for conducting first bandpass filtering on the light with multiple different wavelengths, wherein the first bandpass filtering is used for filtering the light with other wavelengths except the multiple wavelengths.

Further, the system further comprises: a second bandpass filtering device arranged between the sample to be tested and the shooting device and used for conducting second bandpass filtering on the fluorescence, wherein the second bandpass filtering is used for filtering the light with other wavelengths except the wavelengths of the fluorescence.

Further, the system further comprises: a lens arranged between the second bandpass filtering device and the shooting device, wherein a rear focal plane of the lens is located on a photosurface of the shooting device.

Further, the light with multiple different wavelengths emitted by the light excitation module comprises three or more kinds of light.

Further, the system further comprises: a processor for controlling the light excitation module to emit the light with different wavelengths and/or for controlling the shooting device to shoot the fluorescence.

Compared with the prior art, the fluorescent quantitative PCR treatment method and system provided by the present invention have the following beneficial effects:
(1) Through the difference of emission timing of excitation light with different wavelengths and the difference of fluorescence imaging timing on the amplified sample skillfully, the fluorescence emitted from the sample to be tested after the amplification of the sample to be tested excited by the light with different wavelengths reaches the shooting device through the same channel to achieve the purpose of spatial multiplexing of optical channels, thereby implementing the fluorescence calibration of multiple (three or more) wavelengths for the PCR amplification process by a simple optical system, significantly reducing the complexity and the cost of the detection instrument and improving the reliability of the system.
(2) The sensitivity and the accuracy of fluorescent quantitative PCR detection are improved by increasing the number of targets.
(3) A single shooting device is used for achieving real-time imaging of the fluorescence with different wavelengths and reducing the overall cost of the instrument.
(4) Combined with the high integration of the microfluidic chip, the overall instrument is simple in structure, compact and portable, and can realize on-site real-time detection.

The above illustration is only an overview of the technical solution of the present invention. To understand the technical means of the present invention more clearly, the technical solution can be implemented in accordance with contents of the description, and to make above and other purposes, features and advantages of the present invention more apparent and legible, specific embodiments of the present invention are provided below.

### Description of Drawings

One or more embodiments are described exemplarily through pictures in drawings corresponding thereto. The exemplary descriptions are not intended to limit the embodiments. Elements having the same reference number labels in the drawings are expressed as same or similar elements. The diagrams in the drawings do not limit a proportion unless otherwise specified.
Fig. 1 is a flow chart of a fluorescent quantitative PCR treatment method according to embodiments of the present application;
Fig. 2 is a schematic diagram of an optical path system of a channel multiplexing real-time fluorescent quantitative PCR instrument according to embodiments of the present application;
Fig. 3 is a structural diagram of an optical path system of a channel multiplexing real-time fluorescent quantitative PCR instrument according to embodiments of the present application.
Reference Signs: 100-three-color excitation light module, 200-microfluidic chip module, 300-three-channel fluorescence detection module, 400-image processing module, 110-three-color excitation light integrated light source, 120-multi-bandpass filter, 130-reflecting mirror, 210-microfluidic chip, 310-multi-bandpass filter, 320-focusing lens, and 410-camera.

### Detailed Description

To make those skilled in the art better understand the solutions in the present application, the technical solutions in embodiments of the present application will be clearly and fully described below in combination with the drawings in the embodiments of the present application. Apparently, the described embodiments are merely part of the embodiments of the present application, not all of the embodiments. Based on the embodiments in the present application, all other embodiments obtained by those ordinary skilled in the art without contributing creative labor shall belong to the protection scope of the present application.

It should be noted that the terms such as "first", "second", and the like in the Description and Claims as well as Drawings in the present application are used for distinguishing the analogous objects rather than used for describing special order or precedence order. It shall be understood that such data may be exchanged under appropriate circumstances so as to describe the embodiments of the present application. Moreover, terms of "comprise" and "have" as well as any other variant are intended to cover non-exclusive inclusion, for example, processes, methods, systems, products or devices including a series of steps or units are not limited to those steps or units clearly listed, but include other steps or units that are not listed clearly or are inherent to these processes, methods, products or devices.

In the present application, terms such as "upper", "lower", "left", "right", "front", "rear", "top", "bottom", "inner", "outer", "middle", "vertical", "horizontal", "transverse" and "longitudinal" indicate direction or position relationships shown based on the drawings. These terms are mainly intended to better describe the present application and embodiments and not intended to limit that the device, element or component indicated must have a specific direction or must be constructed and operated in a specific direction.

Moreover, part of the above terms may be used for indicating other meanings in addition to the direction or position relationships. For example, the term "up" may also be used for indicating a dependency relationship or connection relationship in some cases. For those ordinary skilled in the art, the specific meanings of these terms in the present application may be understood according to concrete conditions.

It should be noted that the steps shown in the flow chart of the figure can be executed in a computer system such as a set of computer-executable instructions, and although the logical sequence is shown in the flow chart, in some cases, the steps shown or described can be executed in a sequence different from the sequence here.

It should be explained that if there is no conflict, the embodiments in the present application and the features in the embodiments can be mutually combined. The present application will be described in detail below by reference to the drawings and in conjunction with the embodiments.

### Embodiment 1

In the detection instrument of the prior art, if multiple fluorescent PCR detection is used, the fluorescence excited by the light with different wavelengths needs to be detected, and the light of each wavelength is completed by a group of detection devices. For example, the first group of devices is used for detecting the light with the first wavelength, and the second group of detection devices is used for detecting the light with the second wavelength. The number of the groups of detection devices included in the detection instrument is determined by the number of wavelengths of the light that excites the fluorescence to be detected, which not only brings the problem of high cost of the detection instrument, but also uses many elements, resulting in complex optical paths and reduction of stability. Due to the increase of the elements, the performance requirements for key optical elements are increased, and even the performance of the existing optical elements cannot meet the design requirements, which makes it difficult to design a set of complex optical path system to achieve multi-wavelength fluorescence detection. Therefore, the existing detection methods cannot be realized under the condition that there are many wavelengths of light (such as light with three or more colors). In addition, the traditional optical path is complex, has moving components, reduces the stability, and needs to be recalibrated after moving. The systems in the following embodiments can be fixedly integrated, have no moving component, improve the stability, do not need frequent calibration, and have greater advantages in the field of mobile detection and on-site instant detection. The embodiment of the present application is described below.

The method in the present embodiment can detect multicolor light and uses standard PCR characteristics. The standard PCR cycle process comprise three stages: DNA denaturation, annealing and extension; and each of the three stages needs to last for a few seconds respectively. In the entire PCR cycle process, it is not necessary to continuously turn on the light source to excite the sample for fluorescence collection, but only needs to turn on the light source and collect fluorescence signals during a specific period (such as the extension stage). Therefore, the method in the present embodiment uses this characteristic and in the PCR cycle process, the light with multiple (e.g., three) different wavelengths can be sequentially emitted to excite the sample and collect the corresponding fluorescence signals during a specific time period in which the fluorescence signals need to be collected.

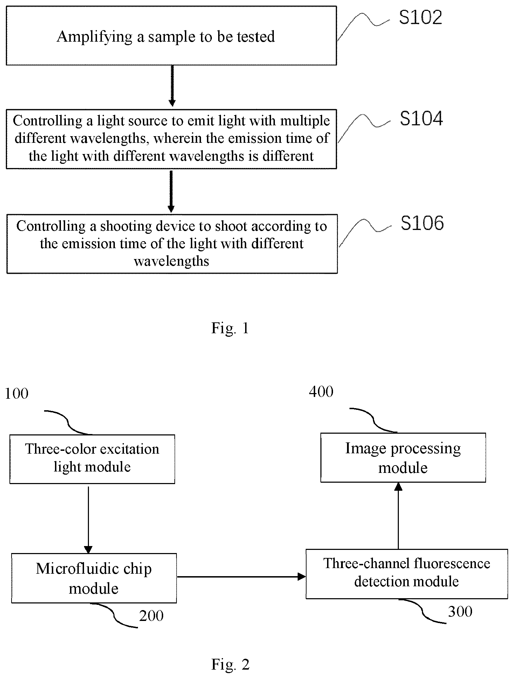

The present embodiment uses a multiplexing idea, that is, the light with different wavelengths is emitted in different time domains, so that the structure of the detection instrument becomes simple and the cost can be reduced. The multiplexing idea can be reflected by a fluorescent quantitative PCR treatment method provided in the present embodiment. Fig. 1 is a flow chart of a fluorescent quantitative PCR treatment system according to embodiments of the present application. As shown in Fig. 1, the flow comprises the following steps:
Step S 102, amplifying a sample to be tested;
Step S104, controlling the light source to emit light with multiple different wavelengths, wherein the emission time of the light with different wavelengths is different;
Step S106, controlling a shooting device to shoot according to the emission time of the light with different wavelengths, wherein objects shot by the shooting device are fluorescence emitted from the sample to be tested after the amplification of the sample to be tested excited by the light with different wavelengths, and the fluorescence reaches the shooting device through the same channel;

As an optional embodiment, an emission time window similar to a frame structure can be defined. For example, the light with the first wavelength is emitted in the first second, the light with the second wavelength is emitted in the 2nd second, and so on. The excitation light in this emission mode is continuously emitted out. There may be interference at the time point of emission wavelength conversion. In addition, camera control also takes time. Considering this problem, an intermittent emission mode can be considered. That is, after the light with the first wavelength is emitted, the light with the second wavelength is emitted at a predetermined time interval, and so on. The interval time can be determined by the difference between the first wavelength and the second wavelength, or a fixed interval time can be used. If the difference between the first wavelength and the second wavelength is greater than a first threshold, a first time interval can be used; and if the difference between the first wavelength and the second wavelength is less than the first threshold, the second time interval is used, and the first time interval is less than the second time interval. As another emission mode that can prevent interference, the waves whose wavelength differences exceed a second threshold can be successively emitted, that is, the light emission time is arranged according to the principle that the difference between adjacent wavelengths of the light emitted is as large as possible.

Step S108, conducting analysis according to images shot by the shooting device. There are many PCR fluorescence analysis methods in the prior art, which will not be described in the present embodiment.

In the above step, one light source can be used for emitting the light with multiple different wavelengths, or a plurality of different light sources can be used for emitting the light with different wavelengths. Regardless of one or more light sources used for emitting the light with different wavelengths, the emission time of the emitted light is different, so that the light can reach the amplified sample to be tested through the same channel. One shooting device is used here, and conducts shooting according to the emission time of the light with different wavelengths, so that the fluorescence emitted from the sample to be tested after the amplification of the sample to be tested excited by the light with each wavelength can be obtained. Because only one optical channel and one shooting device are used for capturing the emitted fluorescence, the use amount of various kinds of hardware is inevitably saved by the measurement of the above steps, thereby reducing the complexity of the hardware, improving the stability of the system and reducing the cost.

In addition, the embodiment of the present invention conducts fluorescence collection by a shooting imaging mode instead of a photodetector, which is conducive to the simultaneous collection of the fluorescence signals of multiple samples on the same microfluidic chip. Taking a six-channel microfluidic chip as an example, a single chip can simultaneously detect 6 samples, and the shooting imaging mode can simultaneously image the fluorescence images of 6 samples. The fluorescence signal intensity of 6 samples can be obtained by only one photo and in combination with data processing. The traditional photodetector method can achieve the fluorescence signal collection of 6 samples by 6 groups of photodetectors, or in the way of moving 1 group of photodetectors, and is complex in the structure. This advantage is more obvious when there are more channels on the microfluidic chip. In addition, the imaging mode is also suitable for imaging of digital PCR chips, and can treat the fluorescence intensity of thousands of micro reaction chambers on digital PCR chips, which cannot be achieved with the photodetector method. Specifically, the shooting device may be a camera, etc.

Because the fluorescence emitted from the sample to be tested after the amplification of the sample to be tested excited by the light with different wavelengths reaches the shooting device through the same channel, in order to reduce the interference between the fluorescence, the sequence and the time of emitting the light with different wavelengths by the light source can be arranged according to the principle that the difference between adjacent wavelengths of the excited fluorescence is as large as possible. This optional mode can reduce the interference without increasing the complexity of the channel.

The light with different wavelengths propagates through the same optical path channel. The interference between the fluorescence is reduced by time sequence emission, and can also be reduced by another optional mode. In this mode, under the same wavelength light source, the fluorescence intensity emitted by the sample excited is simultaneously collected respectively by a matched signal receiving bandwidth and a crosstalk signal receiving bandwidth, and the rule of the fluorescence intensity is counted. In the crosstalk bandwidth, a crosstalk signal is used as background light, and a fluorescence signal threshold is set to eliminate the problem of fluorescence crosstalk. For example, a 475 nm wavelength light source excites two dyes of FAM and HEX at the same time, and their corresponding fluorescence signals are counted respectively. Through statistical analysis, the fluorescence signal threshold is set to eliminate the crosstalk problem.

There are many ways in which the excited fluorescence reaches the shooting device through the same channel. For example, a reflecting surface can be arranged, which forms a certain angle with the propagation direction of the emitted light, that is, the emitted light with different wavelengths is reflected onto the amplified sample to be tested at a first predetermined angle. The excited fluorescence can also be reflected onto the shooting device through a reflecting surface, and the reflecting surface also forms a predetermined angle with the emission direction of the fluorescence, that is, the fluorescence is reflected onto the shooting device at a second predetermined angle. The first predetermined angle may be the same as or different from the second predetermined angle. In an optional embodiment, the angle of the excitation light incident on the sample can be calculated according to the inclination angle of the excitation light source and the distance between the excitation light source and the microfluidic chip, or an appropriate angle can be found experimentally. The two reflecting surfaces may be the same reflecting surface or different reflecting surfaces. In order to save the cost, the same reflecting surface can be used for reflection. In another aspect, considering that the surface of the microfluidic chip used has high reflectivity, the excitation light incident on the sample may produce reflected light, and the reflected light is prone to crosstalk with the excited fluorescence. The angle of the incident excited light and the angle of the emitted fluorescence can be adjusted by arranging the above two reflecting surfaces, so as to effectively prevent crosstalk between the excitation light reflected from the surface of the sample and the generated fluorescence.

In the above embodiments, in order to resist interference, the emission sequence of the light is arranged according to the wavelengths. This mode can reduce the interference between the emitted light. Considering possible interference by stray light in the environment, a bandpass filter can be added, and the bandpass filter can conduct filtering after the light is emitted or before the fluorescence reaches the shooting device, or conduct filtering in two optical paths, which has better effect. That is, after the light source emits the light with multiple different wavelengths, before the light with multiple different wavelengths reaches the sample to be tested, first bandpass filtering is conducted on the light with multiple different wavelengths, wherein the first bandpass filtering is used for filtering the light with other wavelengths except the multiple wavelengths; and/or before the fluorescence reaches the shooting device, second bandpass filtering and/or convergence is conducted on the fluorescence, wherein the second bandpass filtering is used for filtering the light with other wavelengths except the wavelength of the fluorescence, and the convergence is used for focusing the fluorescence to a photosurface of the shooting device. In the preferred embodiment, a lens can also be added to converge the fluorescence, which may improve the shooting effect.

The present embodiment can emit waves with multiple different wavelengths, and especially in the case of three or more different wavelengths of light, the cost advantage and the system stability advantage of the above embodiment will be more obvious.

### Embodiment 2

Based on the principle mentioned in the above method, the present embodiment provides a fluorescent quantitative PCR treatment system, comprising a light excitation module for emitting light with multiple different wavelengths, wherein the emission time of the light with different wavelengths is different; a microfluidic chip module for amplifying the sample to be tested, wherein the fluorescence emitted from the sample to be tested after the amplification of the sample to be tested excited by the light with different wavelengths is transmitted to the shooting device, and the shooting device is used for shooting the fluorescence according to the emission time of the light with different wavelengths; and a single channel, wherein the fluorescence emitted from the sample to be tested after the amplification of the sample to be tested excited by light with different wavelengths reaches the shooting device through the single channel, and the channel can be understood as an optical path and various devices included in the optical path.

The fluorescent quantitative PCR treatment system has simple structure and low cost. The realization device corresponding to the optional embodiment in the above method embodiment is also optional in the present embodiment and may, for example, also include at least one of the following:
a first reflection device for reflecting the light with different wavelengths onto the amplified sample to be tested at the first predetermined angle. For example, the first reflection device may be a reflecting mirror configured to form a certain angle with the light with different wavelengths;
a second reflection device for reflecting the fluorescence onto the shooting device at a second predetermined angle. For example, the second reflection device may be a reflecting mirror configured to form a certain angle with the emitted fluorescence, wherein the first reflection device and the second reflection device are the same reflection device or different reflection devices;
a first bandpass filtering device arranged between the light excitation module and the sample to be tested and used for conducting first bandpass filtering on the light with multiple different wavelengths, wherein the first bandpass filtering is used for filtering the light with other wavelengths except the multiple wavelengths;
a second bandpass filtering device arranged between the sample to be tested and the shooting device and used for conducting second bandpass filtering on the fluorescence, wherein the second bandpass filtering is used for filtering the light with other wavelengths except the wavelengths of the fluorescence;
a lens arranged between the second bandpass filtering device and the shooting device, wherein a rear focal plane of the lens is located on a photosurface of the shooting device;
a processor for controlling the light excitation module to emit the light with different wavelengths and/or for controlling the shooting device to shoot the fluorescence. The processor can be realized in many ways, and any hardware and/or software with control functions can realize the functions of the processor. For example, in the following embodiments, the functions of the processor are realized using a channel multiplexing fluorescence detection module.

Similarly, in the case of three or more different wavelengths of light emitted by the light excitation module in the present embodiment, the cost advantage and the system stability advantage will be more obvious.

### Embodiment 3

Three-color light excitation as an example is described below in combination with a preferred embodiment. In the following preferred embodiment, the three-color excitation light module is the specific realization of the above light excitation module, the bandpass filter is the specific realization of the bandpass filtering device, the reflecting mirror is the specific realization of the reflection device, the focusing lens is the specific realization of the lens, and the camera is the specific realization of the shooting device. In the preferred embodiment, image analysis and the camera can also be regarded as a whole image processing module. The preferred embodiment is described below in combination with the drawings.

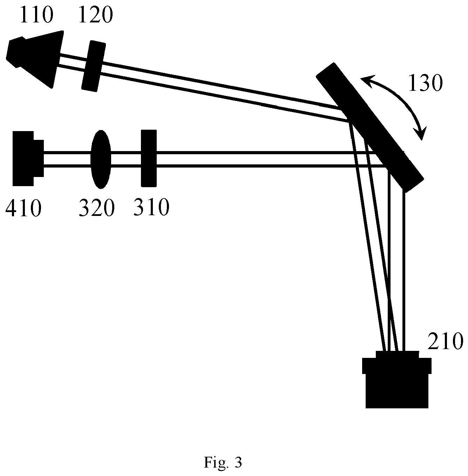

Fig. 2 is a schematic diagram of an optical path system of a channel multiplexing real-time fluorescent quantitative PCR instrument according to the referred embodiment of the present application. Fig. 3 is an optional system architecture diagram based on the schematic diagram of Fig. 2. As shown in Fig. 2 and Fig. 3, the present embodiment provides an optical path system of a single-channel real-time fluorescent quantitative PCR instrument, comprising: a three-color excitation light module 100, a microfluidic chip module 200, a three-channel fluorescence detection module 300 and an image processing module 400.

The three-color excitation light module 100 is used for exciting the sample to be tested so that the sample emits fluorescence, and separating the excitation light emitted by the three-color excitation light module 100 from the fluorescence emitted by the sample to be tested. As shown in Fig. 3, the three-color excitation light module 100 may comprise a three-color excitation light integrated light source 110, a multi-bandpass filter 120 (which can be arranged next to the three-color excitation light integrated light source 110), and a reflecting mirror 130 (which can be arranged between the multi-bandpass filter 120 and the microfluidic chip 210).The wavelengths of three kinds of light emitted by the three-color excitation light integrated light source 110 are different.

The microfluidic chip module 200 is used for amplifying the sample to be tested and enhancing the fluorescence signals. Optionally, the microfluidic chip module 200 comprises a microfluidic chip 210 and a heating and cooling module (not shown in Fig. 3), and the sample to be tested is placed on the heating and cooling module.

The three-channel fluorescence detection module 300 is used for realizing the transmission of fluorescence with different wavelengths in the same channel, and the opening time and frequency of the light source with different wavelengths in the three-color excitation light module 100 and the shooting time and frequency of the camera of the image processing module 400 are controlled so that the fluorescence with different wavelengths emitted from the sample to be tested is sequentially imaged in the same camera. Specifically, as shown in Fig. 3, the three-channel fluorescence detection module 300 may comprise a multi-bandpass filter 310 (which can be arranged between a focusing lens 320 and the reflecting mirror 130) and the focusing lens 320, wherein a rear focal plane of the focusing lens 320 is located on a photosurface of the camera 410.

The image processing module 400 is used for collecting fluorescence signals in real time and analyzing the fluorescence signals. Specifically, the image processing module 400 may comprise the camera 410 and image processing analysis software and hardware.

In the above system, the excitation light with the corresponding wavelength emitted by the three-color excitation light integrated light source 110 is directly reflected through the multi-bandpass filter 120 successively. The reflecting mirror 130 reflects the excitation light beam emitted by the three-color excitation light integrated light source 110 and then the excitation light beam is obliquely incident onto the sample to be tested. Then, the fluorescence excited by the sample to be tested passes through the reflecting mirror 130, the multi-bandpass filter 310 and the focusing lens 320 successively, and then converges on the photosurface of the camera 410, wherein the incident angle at which the excitation light beam is obliquely incident onto the sample to be tested after the reflecting mirror 130 reflects the excitation light beam emitted by the three-color excitation light integrated light source 110 is mainly realized by the adjustment of the reflecting mirror 130, and a specific angle value is determined by the inclination angle of the three-color excitation light integrated light source 110 and the distance between the three-color excitation light integrated light source 110 and the microfluidics chip 210.

The three-color excitation light integrated light source 110 in the present can emit LED light sources with different wavelengths, and can be flexibly selected according to the actual sample to be tested and the fluorescent dye needs. For example, the main wavelengths of the LED light sources are 475 nm, 530 nm and 630 nm, respectively.

At this time, the bandpass ranges of the multi-bandpass filter 120 in the present preferred embodiment are 470-495 nm, 528-538 nm and 628-653 nm, respectively. Other stray light within the ranges of the main wavelengths of the LED light sources is effectively filtered.

The reflecting mirror 130 in the present preferred embodiment is a large-size silvered reflecting mirror. The excitation light beam emitted by the three-color excitation light integrated light source 110 is reflected and then obliquely incident onto the sample to be tested to realize single channel successive excitation.

The bandpass ranges of the multi-bandpass filter 310 in the present preferred embodiment are 498-521 nm, 543-617 nm and 657-751 nm, respectively. The fluorescence emitted by the sample is transmitted through the multi-bandpass filter to realize the separation of the excitation light and the fluorescence and avoid the transmission interference of both. At the same time, the stray light around the central wavelength of the emitted fluorescence can be avoided effectively, and the signal-to-noise ratio and the accuracy of the image can be improved.

The focusing lens 320 in the present preferred embodiment converges the emitted fluorescence transmitted through the multi-bandpass filter 310 so that the width of the fluorescence light beam does not exceed other optical elements to avoid the loss of the fluorescence signals and improve the fluorescence collection efficiency.

Through the above embodiments, multiple excitation light beams are used to make the sample to be tested emit the fluorescence, which improves the accuracy of sample detection. The optical path system of the real-time fluorescent quantitative PCR instrument uses a single camera for achieving real-time imaging of the fluorescence with different wavelengths and reducing the overall cost of the instrument. Combined with high integration and simple structure of the microfluidic chip, the overall instrument is compact and portable. Through rapid image processing, real-time quantitative analysis is realized.

The present application is described with reference to flow charts and/or block diagrams according to the method, device (system) and computer program product in the embodiments of the present application. It should be understood that each flow and/or block in the flow charts and/or block diagrams and a combination of flows and/or blocks in the flow charts and/or block diagrams can be realized through computer program instructions. The computer program instructions can be provided for a processor of a general-purpose computer, a special-purpose computer, an embedded processor, or other programmable data processing devices to generate a machine, so that a device for realizing designated functions in one or more flows of the flow charts and/or one or more blocks of the block diagrams is generated through the instructions executed by the processor of the computer or other programmable data processing devices.

The above only describes the embodiments of the present application and is not intended to limit the present application. For those skilled in the art, various variations and changes can be made to the present application. Any modification, equivalent replacement, improvement, etc. made within the spirit and the principle of the present application shall be included within the scope of claims of the present application.

## Claims

1. A fluorescent quantitative PCR treatment method, comprising:
amplifying a sample to be tested;
controlling a light source to emit light with multiple different wavelengths, wherein the emission time of the light with different wavelengths is different;
controlling a shooting device to shoot according to the emission time of the light with different wavelengths, wherein objects shot by the shooting device are fluorescence emitted from the sample to be tested after the amplification of the sample to be tested excited by the light with different wavelengths, and the fluorescence reaches the shooting device through the same channel;
conducting analysis according to images shot by the shooting device.

2. The fluorescent quantitative PCR treatment method according to claim 1, wherein the light with different wavelengths is reflected onto the amplified sample to be tested at a first predetermined angle; the fluorescence is reflected onto the shooting device at a second predetermined angle; and the first predetermined angle is the same as or different from the second predetermined angle.

3. The fluorescent quantitative PCR treatment method according to claim 2, wherein an incident angle at which the light with different wavelengths is incident into the sample to be tested after reflected is determined according to an inclination angle of the light source and a distance between the light source and a microfluidic chip where the sample to be tested is placed.

4. The fluorescent quantitative PCR treatment method according to any one of claims 1-3, further comprising:
after the light source emits the light with multiple different wavelengths, before the light with multiple different wavelengths reaches the sample to be tested, conducting first bandpass filtering on the light with multiple different wavelengths, wherein the first bandpass filtering is used for filtering the light with other wavelengths except the multiple wavelengths.

5. The fluorescent quantitative PCR treatment method according to any one of claims 1-4, further comprising:
before the fluorescence reaches the shooting device, conducting second bandpass filtering and/or convergence on the fluorescence, wherein the second bandpass filtering is used for filtering the light with other wavelengths except the wavelength of the fluorescence, and the convergence is used for focusing the fluorescence to a photosurface of the shooting device.

6. The fluorescent quantitative PCR treatment method according to any one of claims 1-5, further comprising:
under the same wavelength light source, simultaneously collecting the fluorescence intensity emitted by the sample to be tested excited respectively by a matched signal receiving bandwidth and a crosstalk signal receiving bandwidth, and counting the collected fluorescence intensity to obtain the rule of the fluorescence intensity; and in the crosstalk bandwidth, using a crosstalk signal as background light, and setting a fluorescence signal threshold according to the rule to eliminate the fluorescence crosstalk.

7. The fluorescent quantitative PCR treatment method according to any one of claims 1-6, wherein the light with multiple different wavelengths comprises three or more kinds of light.

8. A fluorescent quantitative PCR treatment system, comprising:
a light excitation module for emitting light with multiple different wavelengths, wherein the emission time of the light with different wavelengths is different;
a microfluidic chip module for amplifying the sample to be tested, wherein the fluorescence emitted from the sample to be tested after the amplification of the sample to be tested excited by the light with different wavelengths is transmitted to the shooting device, and the shooting device is used for shooting the fluorescence according to the emission time of the light with different wavelengths;
a single channel, wherein the fluorescence emitted from the sample to be tested after the amplification of the sample to be tested excited by light with different wavelengths reaches the shooting device through the single channel.

9. The fluorescent quantitative PCR treatment system according to claim 8, further comprising:
a first reflection device for reflecting the light with different wavelengths onto the amplified sample to be tested at a first predetermined angle;
a second reflection device for reflecting the fluorescence onto the shooting device at a second predetermined angle;
wherein the first reflection device and the second reflection device are the same reflection device or different reflection devices.

10. The fluorescent quantitative PCR treatment system according to claim 8 or 9, further comprising:
a first bandpass filtering device arranged between the light excitation module and the sample to be tested and used for conducting first bandpass filtering on the light with multiple different wavelengths, wherein the first bandpass filtering is used for filtering the light with other wavelengths except the multiple wavelengths.

11. The fluorescent quantitative PCR treatment system according to any one of claims 8-10, further comprising:
a second bandpass filtering device arranged between the sample to be tested and the shooting device and used for conducting second bandpass filtering on the fluorescence, wherein the second bandpass filtering is used for filtering the light with other wavelengths except the wavelengths of the fluorescence.

12. The fluorescent quantitative PCR treatment system according to claim 11, further comprising:
a lens arranged between the second bandpass filtering device and the shooting device, wherein a rear focal plane of the lens is located on a photosurface of the shooting device.

13. The fluorescent quantitative PCR treatment system according to any one of claims 8-12, wherein the light with multiple different wavelengths emitted by the light excitation module comprises three or more kinds of light.

14. The fluorescent quantitative PCR treatment system according to any one of claims 8-13, further comprising:
a processor for controlling the light excitation module to emit the light with different wavelengths and/or for controlling the shooting device to shoot the fluorescence.
